# EUROPEAN PATENT SPECIFICATION

(11) **EP 0 456 088 B1**
(45) Date of publication and mention of the grant of the patent: **21.08.1996**
(21) Application number: 91106991.2
(22) Date of filing: 30.04.1991
(51) Int. Cl.: C12Q 1/62, C12Q 1/28, C12N 9/96

(54) **Stabilized uric acid reagent**
Stabilisiertes Harnsäurereagens
Réactif stabilisé pour le dosage de l'acide urique

(30) Priority: 09.05.1990 US 520906
(43) Date of publication of application: 13.11.1991
(73) Proprietor: F. HOFFMANN-LA ROCHE AG, 4002 Basel (CH)
(72) Inventor: Denton, James, Bradley, Montclair, N.J. 07042 (US); Kaufman, Richard Allen, Belleville, N.J. 07109 (US)
(74) Representative: Notegen, Eric-André

(56) References cited:
- EP-A- 0 016 573
- EP-A- 0 036 563
- EP-A- 0 130 708
- FR-A- 2 383 443
- GB-A- 2 014 155
- US-A- 4 250 254
- CHEMICAL ABSTRACTS, vol. 102, no. 5, 04 February 1985, Columbus, OH (US); M.I. SAVENKOVA et al., p. 246, no. 42071d/
- BIOLOGICAL ABSTRACTS, vol. 62, no. 10, 1976, Philadelphia, PA (US); A. WADANO et al., no. 55290/
- ANALYTICAL BIOCHEMISTRY, vol. 134, 1983; M.W. WASHABAUGH et al., pp. 144-152/
- METHODS IN ENZYM. ANALYSIS, vol. 4, 1974; P. SCHEIBE et al., pp. 1951-1958/

## Description

The invention relates to the field of in vitro diagnostics and more particularly to reagents for measuring uric acid in sample body fluids. Such methods typically employ a multiple reagent system containing uricase, peroxidase and an indicator.

Uric acid is the major end product of purine metabolism in man and is one of the components of the non-protein nitrogen fraction in plasma. Serum uric acid levels characteristically are elevated in gout, an inherited disorder involving either uric acid synthesis or excretion. Hyperuricemia is also associated with renal dysfunction, ketoacidosis, and Lesch-Nyhan Syndrome. Diseases that affect nucleoprotein production and metabolism such as leukemia, lymphoma, polycythemia, and various disseminated neoplasms will result in hyperuricemia. It is, therefore, of interest to have accurate, economical, and convenient methods for determining uric acid concentrations in body fluids.

A variety of methods have been used to measure uric acid in blood. Most of the early procedures were colorimetric. Folin and Denis described a method of reacting phosphotungstic acid with uric acid in alkaline solution to form a blue chromophore. [Journal of Biological Chemistry 3: 469 (1972)]. Although there have been numerous applications of the phosphotungstic reduction method, turbidity was often a problem [R. J. Henry, Clinical Chemistry Principles and Technics (1979)]. Kalckar improved the method by the use of uricase to oxidize uric acid to allantoin. The uric acid concentration was determined by absorbance measurement before and after treatment with uricase. [Journal of Biological Chemistry 167:429 (1947)]. Haeckel described an enzyme-coupled approach, using catalase and aldehyde dehydrogenase, to measure the hydrogen peroxide formed by the uricase reaction. The reduction of NAD was used to determine uric acid concentration [Journal of Clinical Chemistry and Biochemistry 14:3 (1976)].

Fossati et al. used peroxidase to detect the hydrogen peroxide formed by the uricase reaction in a modification of the Barham-Trinder reaction [Clinical Chemistry 26:227 (1980)]. The peroxidase in the method of Fossati et al. catalyzed the oxidative coupling of 4-aminoantipyrine with 3,5-dichloro-2-hydroxybenzene sulfonate by reaction with hydrogen peroxide. The interference from bilirubin was minimized by the addition of potassium ferrocyanide.

Other compounds can be used to couple with 4-aminoantipyrine to form chromogens with different wavelengths of detection. Another phenolic compound, 3-hydroxy-2,4,6-tribromobenzoic acid, was described by Trinder and Webster [Annals of Clinical Biochemistry 21:430 (1984)]. Aniline derivatives, including N-ethyl-N-(2-hydroxy-3-sulfopropyl)-m-toluidine (TOOS), have also been synthesized [Chemical Pharmaceutical Bulletin 30:2492 (1982)].

The shelf life of a uric acid reagent is limited by the stability of the enzymes. The uricase-peroxidase reagent requires an intermediate pH between the optimum at 6.5 for peroxidase and 8.5 for uricase. The intermediate pH imparts lower stability to both enzymes in the solution phase. Higher enzyme concentrations have been used also to increase the shelf life of reagents in solution phase, with the resulting disadvantage of higher reagent cost. Separation of some of the destabilizing reagents into a second reagent solution can also increase the shelf life. Destabilizing reagents include phenolic indicators and ferrocyanide. A second reagent solution lowers the convenience of the method and is a potential source of error and waste.

The stability of one component reagents for uric acid varies from one day to three months in different commercial preparations. Fossati et al. (supra) report a stability time of one working day for a one-component reagent based on the uricase-peroxidase reaction. The Uric Acid Liquid Stable Reagent Set of Medical Analysis Systems, Inc. has two liquid reagents containing unspecified preservatives and stabilizers. The combined reagent has a reported stability of 3 months at 2° to 8°C. The problem of lower stability for a single reagent has made multiple reagent solutions or a lyophilized reagent a necessity. A single, ready-to-use reagent would be faster by eliminating time for reagent preparation and more accurate by eliminating volume errors in reconstitution and mixing, as well or more and economical by minimizing waste from the expired combined reagent.

The loss of reagent stability can occur from the degradation of the enzymes or the indicators. The use of polyhydroxy organic compounds for stabilizing a cholesterol reagent [U.S. Patent No. 4,378,429] and an α-amylase reagent [International Patent Publ. WO 89/00600] was described by Modrovich. The polyhydroxyl compounds disclosed included glycerol, ethylene glycol, sorbitol, and propylene glycol.

Improved stabilization of an E. coli dihydroorotase was demonstrated using an ethylene glycol treated with sodium borohydride [Analytical Biochemistry 134, pp. 144-152 (1983)].

Uricase in solution is often sold commercially combined with glycerol [e.g. Sigma Scientific Catalog No. U1377 (50% glycerol)]. The glycerol content in a resulting combined reagent for uric acid, however, will not be high enough to be effective as a stabilizer. [See e.g., Methods of Enzymatic Analysis, Vol. 4, page 1951 (1974); the glycerol content of the described final reagent was less than 0.6 percent. (1974)].

Uricase was stabilized in the lyophilized state with nonionic surfactants, serum albumin and/or basic amino acids [Japanese Patent No. 60,224,499]. A uricase-peroxidase reagent for uric acid was stabilized with EDTA by Marymont and London [The American Journal of Clinical Pathology 42:630 (1964)]. The reagent had uricase, peroxidase, 0-dianisidine hydrochloride, 1mM EDTA, and 0.03% bovine albumin in Tris buffer at pH 7.5. The stability of the refrigerated reagent was several weeks.

The stability of a uric acid reagent was increased by replacing peroxidase with microperoxidase and increasing the pH to 8.2 [European Patent Application Publication No. 195320]. Microperoxidase, a peptide from the protease digestion of cytochrome c, is more stable at alkaline pH than horseradish peroxidase. The uricase is also stabilized with the higher pH buffer, 0.1M borate pH 8.2. The glycerol content is 2.5 percent in the reagent. The reagent is stable for 40 hours at 45°C. [Clinical Chemistry 31:969 (1985)].

The invention provides a reagent which has increased stability in a single solution configuration for use in a method for measuring uric acid concentration in a sample body fluid. More particularly, the stabilized reagent of the present invention comprises a uricase, a peroxidase, an indicator, and as a stabilizer a polyhydroxy nonaromatic alcohol of at least three carbons and at least three hydroxyl groups especially preferred are the polyhydric alcohols glycerol, sorbitol and mannitol. The reagent of the present invention optionally further includes an inert protein and EDTA.

It has been discovered that polyhydroxyl nonaromatic compounds of at least three carbons and three hydroxyl groups such as sorbitol, glycerol and mannitol substantially increase the storage time of a uricase based reagent. The storage time was extended further by the addition of EDTA and an inert protein such as bovine-γ-globulin. The increased thermal stability of the reagent was sufficient to substantially retain enzyme activity when stored at temperatures as high as 45°C for one week.

In order to achieve such increased stability even at raised temperature, it has been found that the hydroxyl, nonaromatic compound must have high purity and be free of destabilizing impurities including metal ions, reducing substances, and protein-denaturing substances. Some compounds, including glycerol, can be distilled multiple times to improve purity. Impurities can also be effectively removed from hydroxyl compounds by treatment with sodium borohydride. The preferred treatment is at alkaline pH for several hours at room temperature. An enhanced reagent stability has been found to result with the borohydride treatment of the polyhydroxyl nonaromatic compounds of the present invention.

It has also been discovered that hydroxyl compounds such as ethylene glycol and propylene glycol do not sufficiently increase the stability of the reagents. The preferred hydroxyl compound is a polyhydric alcohol of at least three carbon atoms and at least three hydroxyl groups.

The preferred hydroxy compound has no absorbance between 400-700 nm, low viscosity, and high solubility. Volume measurement and solution mixing are more efficient for a hydroxy compound with low solution viscosity and high solubility. High solubility is also important for preventing crystallization or precipitation during storage especially at 2°-8°C. Sorbitol because of its low viscosity, high solubility and low cost is a preferred hydroxyl compound.

The polyol may be usefully employed in the present invention over a concentration range of from 10% to a maximum concentration that is limited to that which would detrimentally inhibit the enzymes. The preferred concentration range is 25-50% (wt/vol.%).

EDTA maybe advantageously added to prevent an increase in the blank adsorbance which can result from the nonspecific oxidation of the indicators. The oxidation occurs in the presence of oxygen and catalysts such as metal ions. The preferred EDTA concentration is 0.04-2.0 mM but may be varied considerably from at least 0.01 mM to a maximum concentration of EDTA which is that which would inhibit the activity of the enzymes.

An indicator for detecting hydrogen peroxide from the oxidation of uric acid is another component of the reagent. The preferred indicator is stable in solution and has low bilirubin interference.

Indicators for peroxidase include benzidines, leuco dyes, 4-aminoantipyrine, phenols, naphthols, and aniline derivatives. The preferred indicator is 4-aminoantipyrine and N-ethyl-N-(2-hydroxy-3-sulfopropyl)-m-toluidine (TOOS). The preferred concentration ranges are 0.05 - 10 mM 4-aminoantipyrine and 0.05 - 10 mM TOOS.

Ferrocyanide maybe added to the reagent to minimize interference from bilirubin. However, the presence of metal ions such as ferrocyanide can destabilize the indicator and the enzymes. The stability of the reagent with the hydroxyl stabilizer of the present invention is high enough to allow for the addition of ferrocyanide. The preferred concentration range of ferrocyanide is 1-400 µM, the maximum concentration being that which inhibits the activity of the enzymes.

A nonreactive protein may be added to further increase stability. Nonreactive proteins include serum albumins, globulins, and fibrous proteins. The preferred protein is bovine-γ-globulin at a wt./vol. preferred concentration of 0.05 - 0.3%. Lower concentrations can be useful. The preferred nonreactive protein is free of protease contamination that would cause enzyme degradation.

The enzymes in the reagent must have high purity and solution stability. Microbial uricase has better stability and higher activity at a lower pH range than animal uricase. The preferred uricase is Bacillus fastidiosus or Candida utilis because of low cost and high stability. The enzyme concentration must be high enough for rapid, quantitative reaction with the uric acid at the end of the storage time. The preferred concentration range of Bacillus fastidiosus uricase is 100-800 U/l.

Peroxidase from horseradish is preferred because it is commercially available with high purity and low cost. Enzyme concentration should be high enough for a fast, complete reaction and is preferrably 1,000-10,000 U/l.

Any buffer with sufficient buffering capacity in the pH range from 6.5 to 8.5 is useful. Buffers in this pH range include phosphate, Tris, bis-tris propane, N-tris(hydroxymethyl)methyl-2-aminoethanesulfonic acid (TES) and 3-[N-tris(hydroxymethyl)methylamino]-2-hydroxypropanesulfonic acid (TAPSO). The preferred buffer is phosphate because of its low cost and high stability. The preferred concentration range is 25 to 200 mM phosphate and pH 7.5.

The measurement of uric acid concentration is made with a specified volume and sample body fluid and with a specified volume of reagent. An absorbance reading can be made as soon as possible after mixing and before a significant absorbance change has occurred from metabolism of uric acid to determine a sample blank. A first absorbance reading after 0.5 seconds is appropriate. A second absorbance measurement is made after the absorbance becomes constant which is typically 3.3 minutes at 37°C for a 20 mg/dL uric acid concentration. Typically, the reagent is calibrated with an aqueous or serum solution with a known uric acid concentration.

The volumes for an assay on automated instruments such as the COBAS BIO®, COBAS FARA™, or COBAS MIRA™ (Roche Diagnostic Systems, Inc.) are 15 µl for the sample, 50 µl for the diluent, and 200 ul for the reagent. The volumes of sample and reagent depend on the reaction conditions, the spectrophotometer or other instrument, as well as other variables. Optimization of the sample and reagent volumes for the measurement conditions are within the skilled artisan's capabilities. The concentration of uric acid is determined from the difference between the first and last absorbance reading.

As referred to in the examples which follow, the "factor" for the calibration is the uric acid concentration of the calibrator solution divided by the absorbance difference. The sensitivity is the reciprocal of the factor. After storage of the reagent at 37° or 45°C, the sensitivity decreases and the factor increases.

### Example 1

### Preparation of Reagent

300g of D-sorbitol is added to 300ml of distilled water followed by 1.4g of potassium hydroxide. 0.95g sodium borobydride is added to the solution and allowed to stir overnight at room temperature. 2M phosphoric acid is added to decrease the pH to 6.3. After the sodium borohydride is destroyed, water is added to give a total volume of 900ml and 21.4g dibasic, potassium phosphate anhydrous is added 1.0g methyl 4-hydroxybenzoate(methyl paraben), 0.20g propyl 4-hydroxybenzoate(propyl paraben), 0.37g EDTA, 45mg potassium ferrocyanide, 134mg 4-aminoantipyrine, 0.58g TOOS, 2.0g bovine-γ-globulin is added to the treated sorbitol solution. The pH is adjusted to 7.5 ± 0.1. Then 650U uricase, and 7100U peroxidase are added. The solution volume is increased to 1l with water. After filtration through a 22 µm filter, 0.5g Foamaster FLD (Henkel Corporation) is added.

### Example 2

### Uric Acid Determination on the COBAS BIO, COBAS FARA or COBAS MIRA

To 200µl of the reagent prepared in accordance with Example 1, 15 µl of sample and 50 µl of water diluent are added and mixed. The absorbance readings at 550nm are made at 0.5 seconds and at 200 seconds after mixing of the sample, the diluent, and the reagent. The absorbance difference is converted to a uric acid concentration with a calibration from a calibrator solution with known uric acid concentration.

The following results were obtained on the COBAS MIRA with normal human sera spiked with uric acid. The reference uric acid values were determined at 340 nm on the COBAS MIRA with the ROCHE Reagent for Uric Acid (Roche Diagnostic System, Inc.), a uricase-catalase-aldehyde dehydrogenase method.

| Uric Acid Concentration (mg/dl) | |
|---|---|
| Reference Method | Example 1 Reagent |
| 2.5 | 2.4 |
| 5.3 | 4.8 |
| 10.5 | 9.9 |
| 13.1 | 12.6 |
| 16.5 | 15.8 |
| 20.6 | 19.8 |
| 25.5 | 24.6 |

### Example 3

### Reagent Stability and Sorbitol Concentration

The following reagent was prepared with 20%, 30%, or 40% sorbitol.

The borohydride treatment of sorbitol was made at neutral pH.
20 or 30 or 40g D-sorbitol
95mg sodium borohydride
100mg methyl paraben
20mg propyl paraben
37mg EDTA
100mg bovine-γ-globulin
4.3mg potassium ferrocyanide
12.7mg 4-aminoantipyrine
51mg TOOS
51OU peroxidase
60U uricase
100ml 0.063M phosphate buffer pH 7.5

The reagent was tested with serum samples on the COBAS MIRA after 12 days of storage at 45°C. The factor was determined from the calibrator solution and was used to convert absorbance into uric acid concentration. A high factor is characteristic of low sensitivity for uric acid concentration.

| Reference | Sorbitol 20% | Sorbitol 30% | Sorbitol 40% |
|---|---|---|---|
| Uric Acid Concentration (mg/dl): | | | |
| 4.5 | 3.5 | 4.4 | 4.5 |
| 12.6 | 11.4 | 13.0 | 12.9 |
| 20.3 | 19.0 | 20.1 | 20.8 |
| 24.8 | 22.0 | 23.4 | 24.6 |

| Factor : | | | |
|---|---|---|---|
| 27.2 | 236 | 77.1 | 30.8 |

The uric acid concentration and factor in the first column are representative for all the reagents stored at 4°C. Sorbitol concentration is wt./vol. %

### Example 4

### The Effect of EDTA or Bovine-γ-Globulin on Reagent Stability

The following reagent was prepared without EDTA or without bovine-γ-globulin. The borohydride treatment of sorbitol was made at neutral pH.
- 25g: D-sorbitol
- 79mg: sodium borohydride
- 100mg: methyl paraben
- 20mg: propyl paraben
- 4.2mg: potassium ferrocyanide
- 12.7mg: 4-aminoantipyrine
- 55mg: TOOS
- 37mg: EDTA
- 100mg: bovine-γ-globulin
- 650U: peroxidase
- 60U: uricase
- 100ml: 0.063 M phosphate buffer pH 7.5

The reagent was tested with serum samples on the COBAS MIRA after 7 days of storage at 45°C. The factor was determined from the calibrator solution and was used to convert absorbance into uric acid concentration.

| Reference | Without EDTA | Without Bovine-γ-globulin | With EDTA & with Bovine-γ-globulin |
|---|---|---|---|
| Uric Acid Concentration (mg/dl): | | | |
| 4.6 | 0.2 | 4.0 | 4.4 |
| 8.7 | 8.5 | 8.5 | 8.6 |
| 16.5 | 21.6 | 16.2 | 16.2 |
| 24.4 | 33.2 | 22.9 | 22.8 |

| Factor: | | | |
|---|---|---|---|
| 28.0 | 481 | 70.7 | 34.3 |

The uric acid concentration and factor in the reference column are representative of all the reagents after storage at 4°C.

The highest sensitivity (lowest factor) after storage for one week at 45°C was for reagent with both EDTA and bovine-γ-globulin.

### Example 5

### Effect of Borohydride Treatment of Sorbitol

A comparison of reagent stability was made with no sorbitol and no glycerol, 30% redistilled glycerol, 30% sorbitol with no borohydride treatment, and 30% sorbitol with borohydride treatment at high pH.

0g glycerol or 30g redistilled glycerol or 30g sorbitol, or 30g sorbitol with borohydride treatment (95 mg) at high pH.
- 100 mg: methylparaben
- 20 mg: propylparaben
- 4.4 mg: potassium ferrocyanide
- 37 mg: EDTA
- 100 mg: bovine-γ-globulin
- 13.5 mg: 4-aminoantipyrine
- 58 mg: TOOS
- 710 U.: Peroxidase
- 54 U.: Uricase
- 100 ml: 0.063 M phosphate buffer pH 7.5
- 100.mg: Foamaster FLD

The reagents were tested with serum samples after storage at 4° and 45°C.

| Reference | 0% Glycerol, 0% Sorbitol, 3 days at 45°C | 30% Sorbitol with Borohydride Treatment at High pH 3 days at 45°C |
|---|---|---|
| Uric Acid Concentration (mg/dl): | | |
| 3.3 | 0 | 3.2 |
| 9.2 | 3.7 | 9.0 |
| 14.9 | 11.0 | 14.6 |
| 20.5 | 18.6 | 20.0 |
| 26.1 | 26.4 | 25.1 |

| Factor: | | |
|---|---|---|
| 29 | 606 | 32 |

The numbers in the first column are representative for both reagents at 4°C.

| | Storage for One Week | | | |
|---|---|---|---|---|
| | Storage at 45°C | | | |
| Reference | No Glycerol, No Sorbitol | 30% Glycerol | 30% Sorbitol, No Borohydride Treatment | 30% Sorbitol Borohydride Treatment at High pH |
| Uric Acid Concentration (mg/dl): | | | | |
| 3.3 | 0 | 0.6 | 0 | 2.6 |
| 9.2 | 0 | 7.7 | 6.5 | 9.1 |
| 15.1 | 0.5 | 16.3 | 14.7 | 15.4 |
| 20.6 | 13.7 | 24.6 | 22.5 | 21.6 |
| 26.1 | 27.4 | 32.6 | 30.5 | 27.4 |

| Factor: | | | | |
|---|---|---|---|---|
| 29 | 2077 | 263 | 454 | 91 |

The numbers in the first column are representative for all reagents at 4°C. The most stable reagent has 30% sorbitol with borohydride treatment at high pH.

### Example 6

### Reagent Stability with Borate and Sorbitol

A comparison of reagent stability was made with no sorbitol and no borate, no sorbitol and borate, 30% sorbitol and borate, and 30% sorbitol with borohydride treatment at high pH.
- 0 mg: or 238 mg sodium borate
- 100 mg: methyl paraben
- 20 mg: propyl paraben
- 4.4 mg: potassium ferrocyanide
- 37 mg: EDTA
- 100 mg: bovine-γ-globulin
- 13.5 mg: 4-aminoantipyrine
- 58 mg: TOOS
- 710 U: peroxidase
- 48 U: uricase
- 100 ml: 0.063 M phosphate buffer pH 7.5
- 100 mg: Foamaster FLD

These reagents were tested with serum samples after storage at 4°C and 45°C.

| | Storage for Three Days | | |
|---|---|---|---|
| | Storage at 45°C | | |
| Reference | No Borate, 30% Sorbitol | Borate 30% Sorbitol | 30% Sorbitol, with Borohydride Treatment at High pH |
| Uric Acid Concentration (mg/dl) | | | |
| 3.2 | 1.6 | 2.4 | 3.2 |
| 9.3 | 8.3 | 9.0 | 9.3 |
| 15.0 | 15.6 | 15.4 | 15.2 |
| 20.5 | 22.3 | 21.5 | 20.5 |

| Factor | | | |
|---|---|---|---|
| 30 | 213 | 121 | 35 |

The numbers in the first column are representative for all reagents at 4°C. The reagent with no borate and no sorbitol and the reagent with borate and no sorbitol failed completely after 3 days at 45°C.

| | Storage for Seven Days | | |
|---|---|---|---|
| Storage at 4°C | Storage at 45°C | | |
| Reference | No Borate, 30% Sorbitol | Borate 30% Sorbitol | 30% Sorbitol, with Borohydride Treatment at High pH |
| Uric Acid Concentration (mg/dl): | | | |
| 3.6 | 2.6 | 2.4 | 3.3 |
| 9.1 | 10.2 | 10.3 | 9.6 |
| 14.5 | 17.4 | 18.3 | 15.5 |
| 19.8 | 24.7 | 26.0 | 21.4 |

| Factor | | | |
|---|---|---|---|
| 30 | 347 | 440 | 128 |

The numbers in the first column are representative for all reagents at 4°C. The most stable reagent has 30% sorbitol with borohydride treatment at high pH.

## Claims

1. An aqueous reagent composition for the determination of uric acid in a sample of body fluid which comprises effective amounts of a uricase, a peroxidase, a peroxide indicator, and a polyhydric nonaromatic alcohol of at least three carbons and at least three hydroxyl groups in an amount effective to stabilize said reagent.

2. The composition of claim 1 wherein the polyhydric alcohol is glycerol, sorbitol or mannitol.

3. The composition of claim 1 or claim 2 wherein the polyhydric alcohol is present in a concentration of from 10 to 50% (wt./vol.), preferably from 25 to 50 % (wt./vol.).

4. The composition of any of claims 1 to 3 wherein the polyhydric alcohol is treated to remove destabilizing impurities.

5. The composition of claim 4 wherein the polyhydric alcohol is reacted with a borohydride to remove destabilizing impurities.

6. The composition of any of claims 1 to 5 further comprising EDTA and an inert protein in amounts effective to further stabilize said composition.

7. The composition of claim 6 wherein the polyhydric alcohol is sorbitol in a concentration of from 25 to 50% (wt./vol.) and the inert protein is bovine-γ-globulin in a concentration of from 0.05%-0.3% (wt./vol.).

8. The composition of any of claims 1 to 7 further comprising a ferrocyanide in a concentration effective to inhibit interference with the uric acid determination caused by bilirubin in the sample of body fluid.

## Patentansprüche

1. Wäßrige Reagenszusammensetzung zur Bestimmung von Harnsäure in einer Probe einer Körperflüssigkeit, die wirksame Mengen von Uricase, Peroxidase, eines Peroxidaseindikators und eines mehrwertigen nichtaromatischen Alkohols mit mindestens drei Kohlenstoffatomen und mindestens drei Hydroxylgruppen in einer zur Stabilisierung des Reagenses wirksamen Menge umfaßt.

2. Zusammensetzung nach Anspruch 1, wobei der mehrwertige Alkohol Glycerin, Sorbit oder Mannit ist.

3. Zusammensetzung nach Anspruch 1 oder Anspruch 2, wobei der mehrwertige Alkohol in einer Konzentration von 10 bis 50% (g/v), vorzugsweise 25 bis 50% (g/v) vorhanden ist.

4. Zusammensetzung nach einem der Ansprüche 1 bis 3, wobei der mehrwertige Alkohol zur Entfernung von destabilisierenden Verunreinigungen behandelt ist.

5. Zusammensetzung nach Anspruch 4, wobei der mehrwertige Alkohol zur Entfernung von destabilisierenden Verunreinigungen mit einem Borhydrid umgesetzt worden ist.

6. Zusammensetzung nach einem der Ansprüche 1 bis 5, die des weiteren EDTA und ein inertes Protein in zur weiteren Stabilisierung der Zusammensetzung wirksamen Mengen umfaßt.

7. Zusammensetzung nach Anspruch 6, wobei der mehrwertige Alkohol Sorbit in einer Konzentration von 25 bis 50% (g/v) ist und das inerte Protein Rinder-γ-globulin in einer Konzentration von 0,05 bis 0,3% (g/v) ist.

8. Zusammensetzung nach einem der Ansprüche 1 bis 7, die des weiteren ein Ferrocyanid in einer zur Hemmung der durch Bilirubin in der Probe einer Körperflüssigkeit hervorgerufenen Störung der Harnsäurebestimmung wirksamen Konzentration umfaßt.

## Revendications

1. Composition aqueuse de réactifs pour le dosage de l'acide urique dans un échantillon de fluide corporel qui comprend des quantités efficaces d'uricase, de peroxydase, d'indicateur de peroxyde, et un alcool polyhydrique non aromatique d'au moins trois atomes de carbone et au moins trois groupes hydroxyle en quantité efficace pour stabiliser ledit réactif.

2. Composition selon la revendication 1, caractérisée en ce que l'alcool polyhydrique est le glycérol, le sorbitol ou le mannitol.

3. Composition selon la revendication 1 ou la revendication 2, caractérisée en ce que l'alcool polyhydrique est présent à une concentration de 10 à 50% (P/V), de préférence à une concentration de 25 à 50 % (P/V).

4. Composition selon l'une des revendications 1 à 3, caractérisée en ce qu'on traite l'alcool polyhydrique pour enlever les impuretés déstabilisantes.

5. Composition selon la revendication 4, caractérisée en ce qu'on fait réagir l'alcool polyhydrique avec un borohydrure pour enlever les impuretés déstabilisantes.

6. Composition selon l'une des revendications 1 à 5, qui comprend de plus de l'EDTA et une protéine inerte en quantités efficaces pour une stabilisation supplémentaire.

7. Composition selon la revendication 6, caractérisée en ce que l'acide polyhydrique est le sorbitol à une concentration de 25 à 50% (P/V) et la protéine inerte est la gamma-globuline bovine à une concentration de 0,05%-0,3% (P/V).

8. Composition selon l'une des revendications 1 à 7, qui comprend de plus un ferrocyanure en concentration efficace pour inhiber la perturbation dans le dosage de l'acide urique due à la bilirubine dans l'échantillon de fluide corporel.
